# EUROPEAN PATENT APPLICATION

(11) **EP 4 002 380 A1**
(43) Date of publication of application: **25.05.2022**
(21) Application number: 20208888.6
(22) Date of filing: 20.11.2020
(51) Int. Cl.: G16H 30/20, G16H 50/20

(54) **ASSESSING A REGION OF INTEREST OF A SUBJECT**

(71) Applicant: Koninklijke Philips N.V., 5656 AG Eindhoven (NL)
(72) Inventor: Palero, Jonathan Alambra, 5656 AE Eindhoven (NL); Bourquin, Yannyk Parulian Julian, 5656 AE Eindhoven (NL)
(74) Representative: Philips Intellectual Property & Standards

(57) **Abstract**

According to an aspect, there is provided a computer-implemented method (200) for assessing a region of interest of a subject, the method comprising receiving (202) an image including a portion of a subject; receiving (204) an indication of a location of a region of interest (ROI) on the portion of the subject; receiving (206), from a sensor, sensor data indicative of a measurable parameter of the ROI; receiving (208), from the subject via a user interface, an indication of the nature of the ROI; generating (210) an association between the received sensor data and the nature of the ROI; and providing (212) at least one of the image, the sensor data, the indication of the nature of the ROI and the generated association for further processing.

## Description

### FIELD OF THE INVENTION

The invention relates to assessing a region of interest of a subject and, more specifically, to assessing a region of interest subject using sensor data relating to the region of interest.

### BACKGROUND OF THE INVENTION

Many personal care and personal health activities are performed by people in their own homes. In some cases, such activities may be performed on a person's skin, and may include acquiring data indicative of parameters of the skin for further analysis. Analysis of such data may be performed automatically, using artificial intelligence techniques, and these may include identifying particular areas of the person's skin from which to acquire sensor data, and analysing the acquired data.

Analysis of data is sometimes performed by medical professionals or skincare professionals, who may analyse data acquired in respect of a person's skin or may study an image of the person's skin in order to identify features, such as pimples, skin conditions, ingrowing hairs, moles and the like. However, capturing data and images, and providing them to a specialist for further analysis, can be a time-consuming, complex and expensive process.

Therefore, there is a desire for a system by which analysis of a person's skin can be performed without the need of skin specialists, and without the use of artificial intelligence techniques, which might lack the necessary accuracy.

### SUMMARY OF THE INVENTION

In order to address at least some of the above-mentioned problems associated with existing analysis techniques, the inventors of the present invention have recognised that people are capable of performing some amount of analysis themselves. Accordingly, embodiments of the invention disclosed herein enable a user to provide an annotation relating to a region of interest (e.g. provide an indication of the nature of the region of interest) or a region of their body. For example, a user may position himself or herself in front of a camera to capture an image of the skin on part of their face. The user is able to indicate the location of a region of interest (e.g. a blemish, a mole, a spot, wrinkle, and the like, so that its location can be recorded in an image captured by the camera. In addition, however, the user is able to provide an indication (e.g. an annotation) of the nature of the region of interest, for example by recording what the region of interest is. According to the invention, a parameter of the region of interest is also recorded by acquiring sensor data from a sensor. In this way, an image of the region of interest can be recorded along with an indication of the nature of the region of interest and details of a measurable parameter. Recorded data can be used and processed in various ways, as defined herein.

According to a specific aspect, there is provided a computer-implemented method for assessing a region of interest of a subject, the method comprising: receiving an image including a portion of a subject; receiving an indication of a location of a region of interest (ROI) on the portion of the subject; receiving, from a sensor, sensor data indicative of a measurable parameter of the ROI; receiving, from a user via a user interface, an indication of the nature of the ROI; generating an association between the received sensor data and the nature of the ROI; and providing at least one of the image, the sensor data, the indication of the nature of the ROI and the generated association for further processing.

In some embodiments, the received image may include a first facial landmark feature and a second facial landmark feature. The method may further comprise mapping the received image onto a three-dimensional wire mesh model, based on the position of the first facial landmark feature relative to the second facial landmark feature.

The method may further comprise providing the sensor data and the indication of the nature of the ROI as training data inputs for a predictive model, the predictive model configured to determine the nature of a given ROI based on sensor data indicative of a measurable parameter of the given ROI. In some embodiments, the method may further comprise training the predictive model using the provided sensor data and the provided indication of the nature of the ROI.

The method may, in some embodiments, further comprise providing for presentation on a representation of the subject, an indication of the location of the ROI and the nature of the ROI.

The method may further comprise adjusting, based on the received sensor data, an operating parameter of a personal care device.

In some embodiments, the method may further comprise comparing the received sensor data to a threshold value. The method may comprise, responsive to determining that the received sensor data meets or exceeds the threshold value, generating at least one of: an alert to be provided to the subject; feedback to be provided to a medical professional; a control signal to be provided to a personal care device.

In some embodiments, the method may comprise storing, in a storage device, the received image, the received indication of a location of the ROI, the received sensor data and the received indication of the nature of the ROI.

The method may, in some embodiments, further comprise comparing the received sensor data with previously-stored sensor data. In some embodiments, the method may comprise generating, based on the comparison, at least one of: an alert to be provided to the subject; feedback to be provided to a medical professional; a control signal to be provided to a personal care device; and a treatment profile for the subject.

In some embodiments, the measurable parameter may comprise a measurable skin parameter.

The method may further comprise operating an image capture device to capture the image.

According to a further specific aspect, there is provided computer program product comprising a non-transitory computer-readable medium, the computer-readable medium having computer-readable code embodied therein, the computer-readable code being configured such that, on execution by a suitable computer or processor, the computer or processor is caused to perform the steps of the methods disclosed herein.

According to a further specific aspect, there is provided an apparatus for assessing a region of interest of a subject, the apparatus comprising a processor configured to perform steps of the methods disclosed herein.

According to a further specific aspect, there is provided a system for assessing a region of interest of a user, the system comprising: an image capture device for capturing an image that includes a portion of the user; a region of interest (ROI) selector unit for enabling a user to provide an indication of a location of an ROI on the portion of the subject; a sensor configured to acquire data indicative of a measurable parameter of the ROI; a user interface configured to receive an indication from the user of the nature of the ROI; and a processor configured to: generate an association between the acquired sensor data and the indication of the nature of the ROI; and provide at least one of the image, the sensor data, the indication of the nature of the ROI and the generated association for further processing.

In some embodiments, the ROI selector unit may comprise at least one unit selected from a group comprising: a handheld pointing device; an optical pointing device; a finger-mounted pointing device; a gesture interpretation unit to interpret a gesture made by the user; and an eye gaze interpretation unit to interpret the gaze of the user's eye towards a displayed representation of the ROI.

The sensor may, in some embodiments, comprise a sensor selected from a group comprising: an imaging device; a sensor to measure a reflectance spectrum of the ROI; a moisture sensor; and a sebum measurement device.

These and other aspects will be apparent fro m and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic illustration of an example of a system for assessing a region of interest of a subject;
Fig. 2 is a flowchart of an example of a method of assessing a region of interest of a subject;
Fig. 3 is a flowchart of a further example of a method of assessing a region of interest of a subject;
Fig. 4 is a schematic illustration of a further example of a system for assessing a region of interest of a subject;
Fig. 5 is a schematic illustration of an example of an apparatus for assessing a region of interest of a subject; and
Fig. 6 is a schematic illustration of an example of a processor in communication with a computer-readable medium.

### DETAILED DESCRIPTION OF THE EMBODIMENTS

Embodiments disclosed herein provide a mechanism by which a user is able to provide input regarding a feature of their body such as, for example, input regarding a feature of their skin. Identifying a feature of a person's skin may typically be done by an expert, such as a skin treatment expert, a dermatologist or some other medical professional. However, many features appearing on a person's body will be readily identifiable by the person, and the present invention enables such as identification to be made by an end user, thereby avoiding the need for an expert to spend time and expense studying an image of the person at some later time, and attempting to identify a skin feature.

Examples disclosed herein make reference to regions of interest (ROIs) of a subject. The term "region of interest" (ROI) as used herein is intended to refer to any region of the subject (e.g. a region of a portion of the subject's skin) that contains an area of interest, such as a feature to be monitored, tested or investigated further. Examples of features or ROIs include, but are not limited to, pimples, spots, lesions, areas of acne, pigmented spots, areas of redness, areas of irritated skin cuts, dead skin, scars, moles, wrinkles, hairs, and ingrowing hairs.

It is envisaged that a user (i.e. a subject) may use the system or apparatus disclosed herein in the course of performing a personal care activity, when the user may, for example, be viewing or inspecting their skin (e.g. the skin on their face) in a reflection in a mirror or interactive mirror, or in a live image (e.g. video image) captured by a camera and rendered on a display (e.g. a smartphone or tablet computer). While viewing the reflection or rendered image of their skin, the user positions himself or herself within the field of view of a camera, and indicates, on their skin, the location of an ROI, so that the ROI's position relative to other features of the user (e.g. features of the user's face, such as their nose, eyes and/or mouth) is captured by the camera. In addition, the user provides an input via a user interface that identifies the nature of the ROI, effectively providing a label or annotation that can be associated with the ROI captured by the camera. A measurement of a parameter associated with the ROI is also made using one or more sensors, and the measured parameter may also be associated with the labelled ROI. The data captured by the camera, provided by the user and/or measured using the sensor may then be used for various purposes as discussed herein. For example, in one scenario, the data may be stored and compared with previously-stored data so that any changes in the ROI can be monitored.

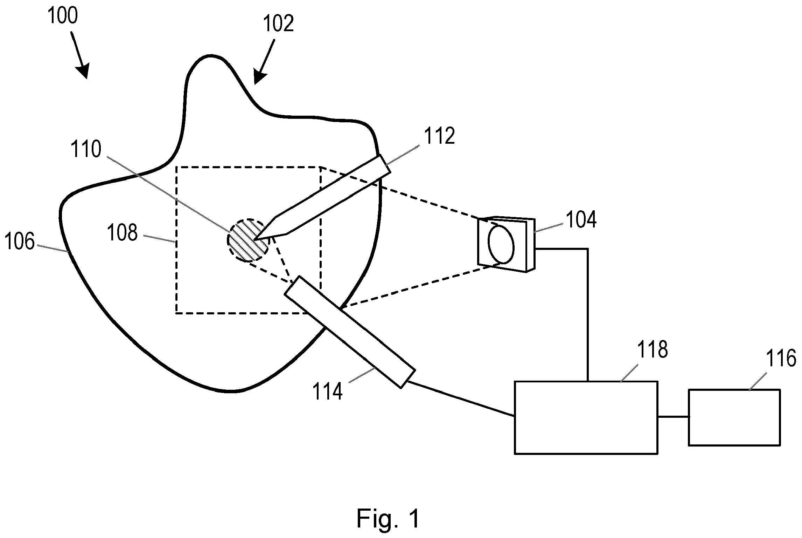

Embodiments are discussed in greater detail below, with reference to the drawings. A first aspect of the invention relates to a system. Fig. 1 is a schematic illustration of an example of a system 100. The system 100 may comprise a system for assessing an ROI of a subject 102 (e.g. a user of the system). The system 100 comprises an image capture device 104 for capturing an image that includes a portion of the subject 102. The image capture device 104 may comprise a two-dimensional image capture device or a three-dimensional image capture device. For example, the image capture device 104 may be configured to capture an image of at least part of the face of the subject 102. Generally, the image captured using the image capture device 104 includes some skin of the subject 102. In the example shown in Fig. 1, a region 106 represents an area of the subject's skin. A dashed box 108 represents the portion of the subject 102 captured using the image capture device 104 in this example. It will be understood that positioning and/or aiming the image capture device 104 differently will result in a different portion of the subject 104 being imaged. In this example, the portion 108 of the subject 102 includes an ROI 110 which the subject 102 intends to image (i.e. capture an image of) and analyse.

The image capture device 104 may comprise any device capable of capturing an image or a series of images (e.g. video footage), and the device may be a stand-alone imaging device or may form part of another device or apparatus. For example, the image capture device 104 may comprise a stand-alone camera or a camera of a smartphone, tablet computer, laptop computer, or interactive mirror. An interactive mirror (sometimes referred to as a smart mirror) is a unit which, in addition to functioning as a mirror to show a user his or her reflection, is also capable of displaying information to the user. Information, such as text, images and videos, may be displayed on a display portion of the interactive mirror which may, for example, be positioned behind a mirrored (or partially-mirrored) panel or a mirrored (or partially-mirrored) surface. In this way, the display screen, or portions thereof, may be visible through the mirror portion, so that a user is able to simultaneously view their reflection and information presented on the display screen.

The system 100 also comprises an ROI selector unit 112 for enabling the subject 102 to provide an indication of a location of an ROI (e.g. the ROI 110) on the portion 108 of the subject. The ROI selector unit 112 can take several different forms and, generally, provides a mechanism that allows the subject 102 to indicate the location or position of the ROI so that the ROI's position can be seen in the captured image. In its simplest sense, according to one example, the ROI selector unit 112 may comprise a pointing device (e.g. a handheld pointing device) that the subject 102 uses to point to the ROI while the image capture device 104 captures an image. This may, in some examples, be achieved by the subject 102 pointing their finger at the ROI. Indicating the location of the ROI may, in some embodiments, be achieved by the subject performing a particular gesture with their hand and/or fingers. In such cases, the ROI selector unit 112 may comprise a gesture interpretation unit to interpret a gesture made by the subject. For example, a user may perform a one-finger pointing gesture to indicate a precise location of an ROI and may perform a two-finger gesture to indicate edges of the ROI (e.g. by marking out a virtual bounding box around the ROI). In some examples, subject 102 may wear a finger-mounted device on their finger or a thumb-mounted device on their thumb (e.g. a thimble-like device or a device that can be strapped or otherwise attached to a digit) that the subject uses to aim or point at the ROI. The ROI selector unit 112 may, in some embodiments, comprise an optical pointer, such as a laser pointer or a device that emits light in a particular waveband or wavelength, such that the light emitted by the device illuminates or is visible on the ROI. In another example, eye tracking or eye gaze tracking may be utilised for selecting or identifying the location of the ROI. For example, the ROI selector unit 112 may comprise a device such as an eye gaze interpretation unit configured to track the subject's eyes or eye gaze in such a way that the subject can indicate the location of the ROI on their skin by directing their gaze to the ROI in their reflection or on a rendered image or representation of them and the ROI on a display. Such a device may be referred to as a virtual pointer.

More generally, the ROI selector unit 112 may comprise at least one unit selected from a group comprising: a handheld pointing device; an optical pointing device; a finger-mounted pointing device; a gesture interpretation unit to interpret a gesture made by the subject; and an eye gaze interpretation unit to interpret the gaze of the subject's eye towards a displayed representation of the ROI.

As noted above, the image capture device 104 may capture individual images (e.g. photographs) or a series of images or frames (e.g. video footage). In examples where the image capture device 104 is configured to capture a series of images, indicating the location of the ROI may be achieved by moving the ROI selector unit 112 over the ROI, to indicate a size (e.g. a width, length or height) or to indicate boundaries of the ROI. For example, the subject 102 may use an ROI selector unit 112 to indicate the location and length of a wrinkle in their forehead by positioning the ROI selector unit at one end of the wrinkle, and moving it along the wrinkle to the other end. In this way, it may be possible to measure or determine from the captured image(s) a length and/or depth of the wrinkle.

In some examples, the image capture device 104 may be configured to capture an image that includes the portion of the subject 102 and also an indication of the location of the ROI on the portion of the subject (e.g. as indicated by the subject using the ROI selector unit 112).

The system 100 further comprises a sensor 114 configured to acquire data indicative of a measurable parameter of the ROI. Several different parameters of the ROI may be measured using the sensor 114. For example, the sensor 114 may be used to acquire data indicative of a measurable skin parameter. The sensor 114 may, in some embodiments, comprise a non-contact sensor or a sensor forming part of a non-contact measurement unit, capable of acquiring data in respect of the ROI without making contact with the ROI itself. In other embodiments, the sensor 114 may comprise a contact sensor or a sensor forming part of a contact measurement unit, configured to acquire data whilst in contact with the ROI.

A sensor 114 configured to acquire data without making contact with the ROI may comprise, for example, an imaging device or imaging sensor such as an RGB (i.e. red, green and blue) camera, a multispectral imaging device, a hyperspectral imaging device or a fluorescence imaging device. The imaging devices/sensors need not acquire an image of the portion of the subject, but may configured to measure a parameter or property of the ROI, such as reflectance. Thus, the sensor 114 may comprise a sensor to measure a reflectance spectrum of the ROI. An RGB camera may be used to measure a color of the ROI, for example to give an indication of redness of skin. In some examples, the image capture device 104 may comprise, or be used as, an RGB camera, such that image data acquired using the image capture device can be analysed to determine the measurable parameter of the ROI. A multispectral imaging device may be used to acquire imaging data (e.g. reflectance data) in wavelengths outside of the visible waveband, for example in the infrared and ultraviolet wavebands, and a hyperspectral imaging device may be used to acquire imaging data (e.g. reflectance data) in wavelengths falling outside of the infrared and ultraviolet wavebands, for example. A fluorescence imaging device may be used to acquire data indicative of a fluorescence of the ROI. Such data may be used to analyse or identify acne, for example.

A sensor 114 configured to acquired data while making contact with the ROI may comprise, for example, a moisture sensor (e.g. a skin moisture sensor) or a sebum measurement device, or sebum metre/sensor, to measure levels of sebum in the ROI (e.g. skin), which may be indicative of an amount of oil in skin.

In some examples, the sensor 114 may form part of the ROI selector unit 112. For example, a handheld unit may comprise one or more sensors 114 for acquiring data indicative of one or more measurable parameters of the ROI. In one example, the sensor 114 may be located at the end of a pen-like unit configured to be held by the subject 102 and touched against the ROI to acquire data. The pen-like unit may also include a laser source to project a laser onto the ROI, thereby also serving as the ROI selector unit 112.

The system 100 also comprises a user interface 116 configured to receive an indication from the subject of the nature of the ROI. The user interface 116 enables a user to provide an annotation or context regarding the ROI and, more specifically, regarding the data acquired using the sensor 114. The user interface 116 may comprise any interface, such as a user input device, that enables the subject to provide an input indicating the nature of the ROI, for example by labelling the ROI with a name or description of what the subject understands it to be. Thus, the user interface 116 may comprise a keyboard for typing the indication of the nature of the ROI, a microphone to enable the subject to speak the indication, a button or a series of buttons each associated with a different indication (e.g. each button being associated with a different type of ROI) enabling the subject to select an indication by pressing one of the buttons, or a touch screen display configured to display a plurality of options or ROI labels from which the subject can select using the touchscreen. In another embodiment, a particular ROI selector unit 112, and/or a particular sensor 114 or a particular attachment for the ROI selector unit and/or the sensor may be associated with and configured for use with a particular ROI, such that use of that particular ROI selector unit, sensor or attachment serves to provide an indication of the nature of the ROI.

In some embodiments, the user interface 116 may comprise a user interface of a separate device, such as a smartphone or a tablet computer while, in other embodiments, the user interface may form a part of or be incorporated into a component of the system 100. For example, the user interface 116 may be provided on the image capture device 104, on the ROI selector unit 112 and/or on the sensor 114, thereby making it easy for the subject 102 to provide the indication of the nature of the ROI at the same time as, or slightly after, providing the indication of the location of the ROI and, possibly, operating the sensor to acquired data from the ROI. Combining features of the system 100 into one component, or at least fewer components, ensures that the subject is able to operate the various components more conveniently.

The system 100 also comprises a processor 118. The processor 118 may be in communication with the image capture device 104, the sensor 114 and the user interface 116. In some embodiments, the processor 118 may also be in communication with the ROI selector unit 112. The processor 118 is configured to generate an association between the acquired sensor data and the indication of the nature of the ROI. For example, the processor 118 may store the data acquired in respect of the ROI together with the indication of the nature of the ROI. In this way, it is possible to recognise any correlations between particular sensor data or particular measurable parameters and particular types of ROI. For example, it may be determined that a high levels of sebum corresponds to acne. Since the indication of the nature of the ROI is provided by the subject 102, the processor 118 (or other systems, such as machine learning models, as discussed below) can, over time, learn which parameters correspond with different types of ROI. In some embodiments, the processor 118 may be configured to generate an association between the acquired sensor data, the indication of the nature of the ROI and an appearance of the ROI obtained from the image captured using the image capture device 104. The generated association may be stored, for example along with the captured image or images, the indication of the location of the ROI, the acquired sensor data and/or the received indication of the nature of the ROI in a storage device (e.g. in a database).

The processor 118 is also configured to provide at least one of: the image(s), the sensor data, the indication of the nature of the ROI and the generated association for further processing. The data may be used in various ways as described in greater detail below. Once the data has been acquired and obtained, it can be used for a range of purposes including, for example, medical analysis, medical or personal care treatment or optimising operating parameters of a device.

In some embodiments, the processor 118 may provide other functionality. For example, the processor 118 may be operatively coupled to one or more components of the system 100 and may be configured to operate one or more of the components. For example, the processor 118 may: control the image capture device 104 to capture an image; operate the sensor 114 to take a measurement of a measurable parameter of the ROI (i.e. record data); operate the ROI selector unit to record the indication of the location of the ROI; and/or operate the user interface to present a series of options to the subject 102 regarding the nature of the ROI for selection by the subject and receive the selection from the user interface.

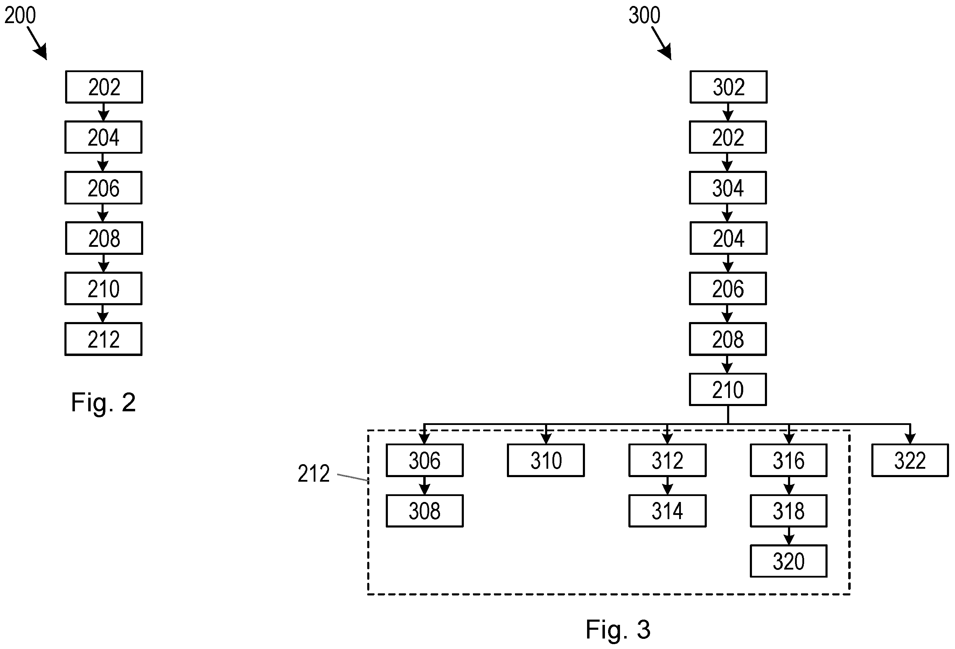

A second aspect of the invention relates to a method. Fig. 2 is a flowchart of an example of a method 200. The method 200, which may comprise a computer-implemented method, may be considered to be a method for assessing a region of interest of a subject. In some embodiments, one or more steps of the method 200 may be performed using a processor, such as the processor 118. The method 200 comprises, at step 202, receiving an image including a portion 108 of the subject 102. The image may be captured using the image capture device 104 and subsequently transferred (e.g. transmitted) to the processor 118. At step 204, the method comprises receiving an indication of a location of an ROI on the portion 108 of the subject 102. The indication of the location of the ROI may be received from the ROI selector unit 112, for example from an indication made by the subject 102 pointing at the ROI. In some embodiments, steps 202 and 204 of the method 200 may be combined, such that step 202 comprises receiving an image including a portion of the subject and an indication of a location of the ROI on the portion of the subject. For example, the image capture device 104 may capture an image of the portion of the subject that includes the ROI and also includes an indication of its location (e.g. by the user pointing to the ROI). In embodiments where steps 202 and 204 are not combined, steps 202 and 204 may be performed concurrently, such that the indication of the location of the ROI is provided by the subject 102 at the same time that the image of the portion of the subject is captured.

The method 200 further comprises, at step 206, receiving, from a sensor 114, sensor data indicative of a measurable parameter of the ROI. The sensor 114 may take the form of any of the sensors discussed herein, and the sensor data acquired using the sensor may be transferred, directly or indirectly, from the sensor to the processor 118.

At step 208, the method 200 comprises receiving, from the subject 102 via a user interface 116, an indication of the nature of the ROI. In other words, the subject 102 provides a label, tag or name for the ROI based on their understanding of what the ROI is. The indication may be provided to the user interface 116 via any of the techniques discussed herein, and the user interface may provide the indication, directly or indirectly, to the processor 118.

The method 200 comprises, at step 210, generating an association between the received sensor data and the nature of the ROI. As discussed above, the association may comprise a stored record including both the sensor data and the nature (e.g. type) of the ROI for a particular ROI. If the method 200 is repeated for multiple ROIs on a subject 102, or for multiple subjects, then it is possible to build up a database of associations, from which it may be possible to determine relationships between certain parameters (i.e. sensor data) and certain types of ROI.

At step 212, the method 200 comprises providing at least one of the image, the sensor data, the indication of the nature of the ROI and the generated association for further processing. Further processing may be performed using the same processor (e.g. the processor 118) used to perform the method 200, or the data may be transferred to or obtained by a different processor or set of processors configured to process the data further. In its simplest sense, the further processing of the data (step 212) may comprise storing at least one of the image, the sensor data, the indication of the nature of the ROI and the generated association in a storage medium, such as a memory.

Fig. 2 is a flowchart of a further example of a method 300. The method 300, which may comprise a computer-implemented method, may also be considered to be a method for assessing a region of interest of a subject. The method 300 may comprise one or more steps of the method 200 discussed above, and one or more steps of the method 300 may be performed using a processor, such as the processor 118 discussed herein.

According to some embodiments, the method 300 may further comprise, at step 302, operating an image capture device (e.g. the image capture device 104) to capture the image. Thus, the processor (e.g. the processor 118) performing steps of the methods 200, 300, may be operatively coupled to the image capture device and may control operation of the image capture device.

Once the image has been captured by the image capture device 104 (step 302) and received (e.g. by the processor 118) at step 202 it may be possible to assess the ROI, and to generate the association between the data and the nature of the ROI, without performing any additional processing. However, in some embodiments, additional processing of the image data captured by the image capture device 104 may be performed in order to improve the quality of the output of the methods and systems disclosed herein. In some embodiments, the received image may include a first facial landmark feature and a second facial landmark feature. Thus, in such embodiments, the image captured using the image capture device 104 comprises an image including at least a portion of the head or face of a subject. Facial landmark features refer to features of a subject's face that may be recognisable by a suitable computer software and which may be used for facial recognition techniques and, more generally, for determining the relative positions of facial features and a general orientation of a subject's head. Facial landmark features may include, for example, the corner and/or edge of a mouth, a nose, an eye, an eyebrow or an ear. Other features of a face or head may also be used as facial landmark features.

The method 300 may comprise, at step 304, mapping the received image onto a three-dimensional mesh model (e.g. a wire mesh model), based on the position of the first facial landmark feature relative to the second facial landmark feature. The three-dimensional wire mesh model may comprise a standard model, for example of a head of a person. The first and second facial landmark features may be used to map the image onto the mesh model to convert the two-dimensional image into a three-dimensional model. In some embodiments, the image capture device 104 may be configured to capture a three-dimensional image of the portion of the subject 102 and, in such cases, mapping of the image onto a mesh model may be simplified or, in some cases, not needed. While to facial landmark features may be required in order to determine a distance between the features and, therefore, to predict the distance between other facial landmark features, it will be understood that a more accurate mapping of the image onto the three-dimensional model may be achieved if a large number of facial landmark features are used. Therefore, the subject 102 may be encouraged to move into a position that enables the image capture device 104 to capture as facial landmark features as possible. A result of the mapping step (i.e. step 304) is the creation of a reference facial map, and the location of the ROI may be defined relative to the reference facial map. In some embodiments, a previously-mapped three-dimensional model may be used or updated using the newly-captured image.

As noted above, at least one of the image, the sensor data, the indication of the nature of the ROI and the generated association is provided (at step 212) for further processing, and various processing techniques that may be implemented are discussed below. One or more of the processing techniques discussed below may be performed individually or concurrently, and the various techniques are depicted in Fig. 3 as alternatives within a dashed box representing the step 212.

At step 306, the method 300 may comprise providing the sensor data and the indication of the nature of the ROI as training data inputs for a predictive model. The predictive model may be configured to determine the nature of a given ROI based on sensor data indicative of a measurable parameter of the given ROI. Such a predictive model may comprise a machine learning model or algorithm including for example artificial neural networks, deep neural networks, regression trees, classifiers, and the like. In some embodiments, the data provided at step 306 may be used as initial training data to train the predictive model while, in other embodiments, the data may be used to re-train a previously-trained predictive model in order to improve the model and thus improve the model's outputs (e.g. its accuracy). Thus, step 308 of the method 300 may comprise training the predictive model using the provided sensor data and the provided indication of the nature of the ROI.

The method 300 may comprise, at step 310, providing for presentation on a representation of the subject 102, an indication of the location of the ROI and the nature of the ROI. For example, if the subject 102 is using an interactive mirror, then the subject's reflection may comprise the representation, and the interactive mirror may present the ROI on the reflection of the subject's face, with a label indicating what the ROI is (e.g. a spot, acne, a wrinkle, or the like). In another embodiment, the representation of the subject may comprise a computer-generated representation, such as a realistic image (e.g. a photograph) of the subject 102, a generic image or diagram of a face, or an avatar. The representation of the subject 102 may be presented or displayed to the subject on a different device, such as a smartphone or tablet computer or on some other computing device, such as a wearable device (e.g. a smartwatch). By displaying the ROI (e.g. the location of the ROI and the nature of the ROI) on the representation of the subject 102, the subject is able to see which ROIs they have already identified, and have therefore been recorded (e.g. with an association having been generated).

At step 312, the method 300 may comprise comparing the received sensor data to a threshold value. Each different type of sensor may have a respective threshold value against which data is to be compared. In some embodiments, the threshold value may vary depending on details of the subject (e.g. the subject's age, gender, medical history, and the like). Threshold values may be stored in a storage device (e.g. a memory) associated with the system 100 and accessible by the processor 118 to enable a comparison to be made. In some embodiments, a threshold value may be selected or set based on and accepted health the limit for the value. For example, if a sensor 114 were to detect that the redness of an ROI exceeded a defined threshold level of redness, then this may be indicative of a medical condition requiring medical intervention. The method 300 may comprise, at step 314, responsive to determining that the received sensor data meets or exceeds the threshold value, generating at least one of: an alert to be provided to the subject; feedback to be provided to a medical professional; a control signal to be provided to a personal care device. An alert to be provided to the subject 102 may, for example, comprise a notification or warning that a particular parameter (i.e. the parameter measured using the sensor) is at such a level that the ROI should be monitored. In some embodiments, the alert for the subject 102 may comprise a notification that the parameter is at a suitable level for applying treatment, or for applying the next course of treatment, in a treatment plan. The alert may, therefore, comprise a reminder to the subject 102 to perform the next treatment of a course of treatment if it is determined that the parameter value is at the required level (i.e. meets the threshold). Feedback may be generated for provision to a medical professional if it is detected that the measurable parameter has exceeded a level where there may be cause for concern. For example, if the sensor data is such that there is a chance that the ROI could be exhibiting signs of a particular medical condition (e.g. cancer) then feedback (e.g. an alert) may be generated and provided to a medical professional.

In some examples, the system 100 may be used in conjunction with a personal care device, such as a skin treatment device. The parameter of the ROI measured using the sensor may result from the application of treatment using the skin treatment device. It may be determined that, due to the level of the parameter, an adjustment should be made to the skin treatment device (e.g. to reduce a power output of the device). In such cases, step 314 may involve generating a control signal to be provided to the device to take appropriate action.

The method 300 may comprise, at step 316, storing, in a storage device, the received image, the received indication of a location of the ROI, the received sensor data and the received indication of the nature of the ROI. As noted above, such a storage device (e.g. a memory) may form part of the system 100 and/or may be accessible by the processor (e.g. the processor 118) performing the method 200, 300.

At step 318, the method 300 may comprise comparing the received sensor data with previously-stored sensor data. The previously-stored sensor data may comprise that the data stored in the storage device at step 316, for example. The method 300 may comprise, at step 320, generating, based on the comparison, at least one of: an alert to be provided to the subject; feedback to be provided to a medical professional; a control signal to be provided to a personal care device; and a treatment profile for the subject. As discussed above, the alert for the subject, the feedback for the medical professional and the control signal for the personal care device may be generated if it is determined that the new sensor data differs sufficiently from the previously-stored sensor data that action should be taken. For example, the comparison of data may reveal that a particular parameter of the ROI has worsened significantly and, therefore, that medical attention is required. Conversely, in an example where the subject 102 is applying treatment to the ROI, the data comparison may reveal that a particular parameter of the ROI has improved (e.g. a level of redness has reduced) and, therefore, a control signal to be provided to a personal care device (e.g. the treatment device being used in the course of treatment) to reduce the effect of the treatment device (e.g. by reducing the power output of the device).

In one embodiment, the comparison 318 may be used to generate a treatment profile for the subject 102. Such a profile may be specific to the subject 102 and may include details relating to the subject's skin and, in particular, to the measurable parameter(s) of the subject's skin. In one such example, the comparison may reveal that the subject's skin has become more oily, and this may be recorded in a treatment profile used by a skin treatment expert or beauty therapist when developing a treatment program involving topicals (e.g. creams and ointments).

In some embodiments, the method 300 may further comprise, at step 322, adjusting, based on the received sensor data, an operating parameter of a personal care device (e.g. a personal treatment device). The adjusting performed at step 322 may be performed separately from the further processing of step 212 or as part of step 212. Thus, a personal care device may be adjusted on the basis of the received centre data directly, without comparing the received sensor data with previously-stored data or with a threshold value.

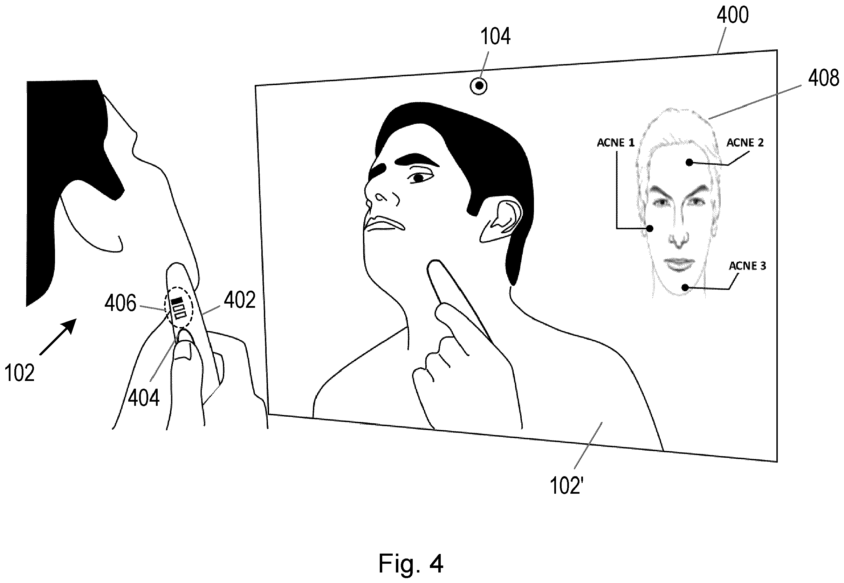

Fig. 4 is an illustration of an example of the system 100 of Fig. 1 in use. In the example shown, the subject 102 is viewing their reflection 102' in an interactive mirror 400. The interactive mirror 400 includes a camera (i.e. an image capture device) 104 and a processor (not shown), such as the processor 118. In this example, the subject 102 holds a handheld device 402 which functions as both an ROI selector unit 112 and a sensor 114. The subject 102 is able to point the handheld device 402 at the ROI in order to indicate the location of the ROI on the portion of their skin. In another embodiment, the subject 102 may operate a laser pointer built into the handheld device 402 to project a laser onto the ROI (e.g. into the centre of the ROI). The handheld device 402 includes a button 404 which the subject 102 is able to press in order to provide an indication of the nature of the ROI. In this example, the handheld device 402 also includes a series of lights 406, and each light may represent a different type of ROI. Pressing the button 404 enables the subject 102 to cycle through the various types of ROI until the desired light is illuminated, which corresponds to the type of ROI that the subject is identifying. For example, the handheld device 402 may include a first light corresponding to acne, a second light corresponding to a pigmented spot, and a third light corresponding to an area of irritated skin. Once the subject 102 has indicated the location of the ROI and indicate the nature of the ROI using the button 404, the indications may be provided to the processor (not shown) in order for the association to be generated.

In the example shown in Fig. 4, a representation 408 of the subject 102 is displayed on the interactive mirror 400 along with an indication of various ROIs (e.g. labelled 'ACNE 1', 'ACNE 2' and 'ACNE 3') which may, for example, have been previously-recorded by the subject. In this way, the subject 102 is able to see which ROIs they have identified.

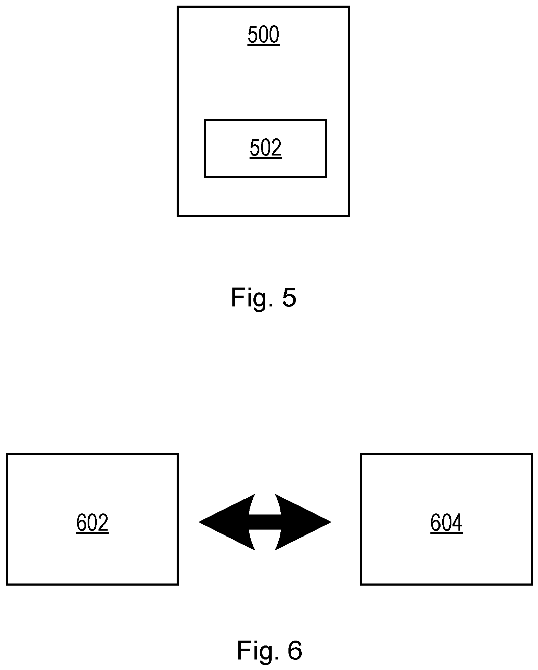

A third aspect of the invention relates to an apparatus. Fig. 5 is a schematic illustration of an example of an apparatus 500. The apparatus 500 may be referred to as an apparatus for assessing a region of interest of a subject 102. The apparatus 500 comprises a processor 502 configured to perform one or more steps of the methods 200, 300 discussed herein. Thus, the processor 502 may be configured to receive an image including a portion of a subject; receive an indication of a location of a region of interest (ROI) on the portion of the subject; receive, from a sensor, sensor data indicative of a measurable parameter of the ROI; receive, from the subject via a user interface, an indication of the nature of the ROI; generate an association between the received sensor data and the nature of the ROI; and provide at least one of the image, the sensor data, the indication of the nature of the ROI and the generated association for further processing. In some embodiments, the processor 502 may comprise, or be similar to, the processor 118 discussed above. The apparatus 500 may comprise or form part of a computing device, such as a desktop computer, a laptop computer, a tablet computer, a smartphone, a wearable device such as a smartwatch, an interactive mirror or a server. In some embodiments, the apparatus 500 may comprise one or more of the components 104, 112, 114, 116 of the system 100 discussed above.

A fourth aspect of the invention relates to a computer program product. Fig. 6 is a schematic illustration of an example of a processor 602 in communication with a computer-readable medium 604. According to embodiments, a computer program product comprises a non-transitory computer-readable medium 604, the computer-readable medium having computer-readable code embodied therein, the computer-readable code being configured such that, on execution by a suitable computer or processor 602, the computer or processor is caused to perform the steps of the methods 200, 300 discussed herein. Thus, the computer-readable medium 604 may have computer-readable code embodied therein which is configured such that, on execution by a suitable computer or processor, the computer or processor is caused to receive an image including a portion of a subject; receive an indication of a location of a region of interest (ROI) on the portion of the subject; receive, from a sensor, sensor data indicative of a measurable parameter of the ROI; receive, from the subject via a user interface, an indication of the nature of the ROI; generate an association between the received sensor data and the nature of the ROI; and provide at least one of the image, the sensor data, the indication of the nature of the ROI and the generated association for further processing.

Thus, embodiments of the present invention provide a mechanism by which a subject is able to provide annotations to indicate the nature of an ROI themselves. This makes use of the understanding that a subject knows what particular features of their skin are, and enables them to label or tag them as such while an image of their skin is captured for future analysis. At the same time, may measurable parameter of the subject's body (e.g. the skin) is measured using a sensor, and this measurement is associated with the ROI such that the recorded data can be used for various subsequent processing techniques. The invention therefore helps to reduce the amount of subsequent processing required in respect of the captured image by reducing the need for a medical expert to analyse the image and provide their own indication of the nature of the ROI. This not only reduces the amount of processing required, but also reduces the time between capturing the image and labelling the ROI. This can be particularly advantageous if the sensor data acquired in respect of the ROI indicates that the ROI requires urgent medical attention.

The processor 118, 502, 602 can comprise one or more processors, processing units, multi-core processors or modules that are configured or programmed to control the apparatus 500 in the manner described herein. In particular implementations, the processor 118, 502, 602 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (200) for assessing a region of interest of a subject, the method comprising:
receiving (202) an image including a portion of a subject;
receiving (204) an indication of a location of a region of interest (ROI) on the portion of the subject;
receiving (206), from a sensor, sensor data indicative of a measurable parameter of the ROI;
receiving (208), from the subject via a user interface, an indication of the nature of the ROI;
generating (210) an association between the received sensor data and the nature of the ROI; and
providing (212) at least one of the image, the sensor data, the indication of the nature of the ROI and the generated association for further processing.

2. A computer-implemented method (200, 300) according to claim 1, wherein the received image includes a first facial landmark feature and a second facial landmark feature, and wherein the method further comprises:
mapping (304) the received image onto a three-dimensional wire mesh model, based on the position of the first facial landmark feature relative to the second facial landmark feature.

3. A computer-implemented method (200, 300) according to claim 1 or claim 2, further comprising:
providing (306) the sensor data and the indication of the nature of the ROI as training data inputs for a predictive model, the predictive model configured to determine the nature of a given ROI based on sensor data indicative of a measurable parameter of the given ROI; and
training (308) the predictive model using the provided sensor data and the provided indication of the nature of the ROI.

4. A computer-implemented method (200, 300) according to any of the preceding claims, further comprising:
providing for presentation on a representation of the subject, an indication of the location of the ROI and the nature of the ROI.

5. A computer-implemented method (200, 300) according to any of the preceding claims, further comprising:
adjusting (322), based on the received sensor data, an operating parameter of a personal care device.

6. A computer-implemented method (200, 300) according to any of the preceding claims, further comprising:
comparing (312) the received sensor data to a threshold value; and
responsive (314) to determining that the received sensor data meets or exceeds the threshold value, generating at least one of: an alert to be provided to the subject; feedback to be provided to a medical professional; a control signal to be provided to a personal care device.

7. A computer-implemented method (200, 300) according to any of the preceding claims, further comprising:
storing (316), in a storage device, the received image, the received indication of a location of the ROI, the received sensor data and the received indication of the nature of the ROI.

8. A computer-implemented method (200, 300) according to claim 7, further comprising:
comparing (318) the received sensor data with previously-stored sensor data; and
generating (320), based on the comparison, at least one of: an alert to be provided to the subject; feedback to be provided to a medical professional; a control signal to be provided to a personal care device; and a treatment profile for the subject.

9. A computer-implemented method (200, 300) according to any of the preceding claims, wherein the measurable parameter comprises a measurable skin parameter.

10. A computer-implemented method (200, 300) according to any of the preceding claims, further comprising:
operating (302) an image capture device to capture the image.

11. A computer program product comprising a non-transitory computer-readable medium, the computer-readable medium (604) having computer-readable code embodied therein, the computer-readable code being configured such that, on execution by a suitable computer or processor (602), the computer or processor is caused to perform the method of any of the preceding claims.

12. An apparatus (500) for assessing a region of interest of a subject, the apparatus comprising a processor (502) configured to perform the steps of any of claims 1 to 10.

13. A system (100) for assessing a region of interest of a subject, the system comprising:
an image capture device (102) for capturing an image that includes a portion of the subject;
a region of interest (ROI) selector unit (112) for enabling the subject to provide an indication of a location of an ROI on the portion of the subject;
a sensor (114) configured to acquire data indicative of a measurable parameter of the ROI;
a user interface (116) configured to receive an indication from the subject of the nature of the ROI; and
a processor (118) configured to:
generate an association between the acquired sensor data and the indication of the nature of the ROI; and
provide at least one of the image, the sensor data, the indication of the nature of the ROI and the generated association for further processing.

14. A system (100) according to claim 13, wherein the ROI selector unit comprises at least one unit selected from a group comprising: a handheld pointing device; an optical pointing device; a finger-mounted pointing device; a gesture interpretation unit to interpret a gesture made by the subject; and an eye gaze interpretation unit to interpret the gaze of the subject's eye towards a displayed representation of the ROI.

15. A system (100) according to claim 13 or claim 14, wherein the sensor comprises a sensor selected from a group comprising: an imaging device; a sensor to measure a reflectance spectrum of the ROI; a moisture sensor; and a sebum measurement device.
